# EUROPEAN PATENT SPECIFICATION

(11) **EP 1 927 658 B1**
(45) Date of publication and mention of the grant of the patent: **10.10.2012**
(21) Application number: 08004770.7
(22) Date of filing: 21.06.2005
(51) Int. Cl.: C12N 9/74, C12N 9/96

(54) **Thrombin compositions**
Thrombinzusammensetzungen
Compositions de thrombine

(30) Priority: 22.06.2004 US 581824 P
(43) Date of publication of application: 04.06.2008
(62) Divisional of application: 05766031.8
(73) Proprietor: Zymogenetics, Inc., Princeton, NJ 08543 (US)
(72) Inventor: Jiang, Shan, Sammamish, WA 98075 (US); Senderoff, Richard I., Edmonds, WA 98026 (US); Meyer, Jeffrey D., Lake Forest Park, WA 98155 (US)
(74) Representative: Tombling, Adrian George

(56) References cited:
- WO-A-01/23001
- US-A- 4 923 815
- KOLODZEISKAYA M.V. AND CHERNYSHENKO T.M.: "Human thrombin: enzymatic properties, stability and standardization of preparation" UKRAINSKII BIOKHIMICHESKII ZHURNAL, vol. 74, no. 5, September 2002 (2002-09), - October 2002 (2002-10) pages 27-33, XP008053548

## Description

### BACKGROUND OF THE INVENTION

Formulation of pharmaceutical proteins presents significant challenges. Proteins possess multiple functional groups in addition to three-dimensional structure; degradation therefore proceeds via both chemical (modifications involving bond formation or cleavage) and physical (denaturation, aggregation, adsorption, precipitation) pathways. Since each protein embodies a unique combination of amino acid sequence, isoelectric point, and other determinants, its response to changes in solution conditions is unpredictable, and must be determined on a case-by-case basis. Attempts to prevent one form of degradation often increase the rate of another.

Degradation of proteins can be greatly reduced or avoided by lyophilization. However, lyophilization is time-consuming and costly, and can cause protein denaturation and aggregation if appropriate excipients are not included. As with solution formulation, stabilization of lyophilized proteins must be dealt with on an individual basis.

In the case of thrombin, sodium chloride can be used to maintain stability during purification and storage by reducing aggregation and precipitation. However, sodium chloride is a problematic excipient in lyophilized formulations because it lowers glass transition temperature, thereby necessitating low primary temperatures and long cycle times. In addition, thrombin must be protected from unfolding and aggregation within the lyophilized composition.

The ideal formulation will combine stability during lyophilization, as well as long-term storage and shipment with ease of handling and use.

US-A-4923815 describes a stable dried thrombin composition, comprising saccharides. Among the saccharides concerned, sucrose and mannitol are mentioned.

### DESCRIPTION OF THE INVENTION

Within one aspect of the invention there is provided a composition consisting essentially of 0.1 mg/mL to 5.0 mg/mL thrombin, 2% to 4% (w/v) sucrose, 3.5% to 5% (w/v) mannitol, 50 mM to 300 mM NaCl, 0-5mM CaCl₂, 0.03% to 1% (w/v) of a surfactant or high-molecular-weight polyethylene glycol, and a physiologically acceptable buffer, in aqueous solution at pH 5.7 - 7.4, wherein concentration of the buffer is selected to provide approximately physiological pH upon application of the composition in a surgical setting and wherein the ratio of mannitol:sucrose is greater than 1:1 but not greater than 2.5:1 (w/w). Within one embodiment of the invention the ratio of mannitol to sucrose is 1.33:1 (w/w). Within other embodiments of the invention the molar ratio of sucrose:thrombin is at least 700:1 or at least 2000:1. Within further embodiments of the invention the thrombin is human thrombin, including, for example, recombinant human thrombin. Within still other embodiments of the invention, the thrombin concentration is 0.5 - 3.0 mg/ml or the thrombin concentration is 1 mg/ml. Within other embodiments of the invention, the sucrose concentration is 3% (w/v) and/or the mannitol concentration is 4% (w/v). Within a further embodiment the surfactant or high-molecular-weight polyethylene glycol is PEG 3350. Within a related embodiment the surfactant or high-molecular-weight polyethylene glycol is PEG 3350 at a concentration of 0.1% (w/v). Within additional embodiments, the pH of the composition is 6.0. Within further embodiments the buffer is selected from the group consisting of histidine, citrate, phosphate, Tris, and succinate buffers. Within related embodiments the buffer is histidine buffer, such as 2.0-10 mM histidine buffer or 5 mM histidine buffer. One such composition of the present invention consists essentially of 0.1 mg/mL to 5.0 mg/mL thrombin, 2% to 4% (w/v) sucrose, 3.5% to 5% (w/v) mannitol, 100 mM to 200 mM NaCl, 1 mM to 5 mM CaCl₂, 0.03% to 1% (w/v) PEG3350, and 2 mM to 10 mM histidine in aqueous solution at pH 5.5 - 6.5, wherein the ratio of mannitol: sucrose is greater than 1:1 but not greater than 2.5:1 (w/w). A second such composition of the invention consists essentially of 1 mg/ml thrombin, 3% (w/v) sucrose, 4% (w/v) mannitol, 4 mM CaCl₂, 0.1% (w/v) PEG3350, 150 mM NaCl, and 5 mM histidine in aqueous solution at pH 6.0.

Within a second aspect of the invention there is provided a method of preparing a lyophilized thrombin composition comprising (a) providing an aqueous, thrombin-containing composition as disclosed above and (b) lyophilizing the aqueous composition to form a lyophilized thrombin composition.

Within a third aspect of the invention there is provided a lyophilized thrombin composition prepared by the method disclosed above. Within one embodiment of the invention the lyophilized composition is contained in a sealed container having a label affixed to an exterior surface thereof. Within an additional embodiment of the invention the composition in the sealed container contains from 2,500 to 20,000 NIH units of thrombin. Within a related embodiment of the invention the composition is provided in the form of a kit comprising a sealed container as disclosed above and a second sealed container containing a diluent such as, for example, normal saline.

These and other aspects of the invention will become evident upon reference to the following detailed description of the invention and the attached drawings. In the drawings:
Fig. 1 illustrates the analysis of pre- and post-lyophilization samples of certain thrombin formulations by Right Angle Light Scattering (RALS).
Fig. 2 illustrates the analysis of certain thrombin formulations by RALS following storage for thirteen weeks.
Fig. 3 illustrates the analysis of certain thrombin formulations by RALS following storage for six months.
Fig. 4 illustrates the analysis of thrombin secondary structure by Fourier transform infrared spectroscopy using four formulations of recombinant human thrombin (labeled A, B, C, and D).
Fig. 5 illustrates the analysis of thrombin secondary structure by Fourier transform infrared spectroscopy using five formulations of recombinant human thrombin (labeled T, A, B; C, and D).

Numerical ranges (e.g., "from X to Y") include the endpoints unless otherwise specified.

The present invention provides stabilized thrombin compositions, including lyophilized compositions. As used herein, "thrombin" denotes the activated enzyme, also known as α-thrombin, which results from the proteolytic cleavage of prothrombin (factor II). As disclosed below, thrombin can be prepared by a variety of methods known in the art, and the term "thrombin" is not intended to imply a particular method of production. Human thrombin is a 295 amino acid protein composed of two polypeptide chains joined by a disulfide bond. Both human and non-human thrombins can be used within the present invention. Thrombin is used medically as a hemostatic agent and as a component of tissue adhesives.

Human and non-human (e.g., bovine) thrombins are prepared according to methods known in the art. Purification of thrombin from plasma is disclosed by, for example, Bui-Khac et al., U.S. Patent No. 5,981,254. Purification of thrombin from plasma fractions, such as Cohn fraction III, is disclosed by Fenton et al., J. Biol. Chem. 252:3587-3598, 1977. Recombinant thrombin can be prepared from a prethrombin precursor by activation with a snake venom activator as disclosed in U.S. Patent No. 5,476,777. Suitable venom activators include ecarin and prothrombin activator from *Oxyuranus scutellatus.*

Within the present invention, thrombin is formulated in a weakly buffered, aqueous solution containing sucrose, mannitol, sodium chloride, a surfactant or high molecular weight polyethylene glycol (HMW-PEG), and, optionally, calcium chloride. Concentration ranges of these components are shown in Table 1. Concentrations expressed as percent are on a weight-to-volume basis.

**Table 1**

| | |
|---|---|
| thrombin | 0.01-5.0 mg/mL |
| sucrose | 2% - 4% |
| mannitol | 3.5%-5% |
| NaCl | 50-300 mM |
| surfactant or HMW-PEG | 0.03%-1% |
| CaCl₂ | 0-5 mM |
| pH | 5.7-7.4 |

The concentration of thrombin within the formulation of the present invention can be varied depending on the intended use, including the desired concentration of the reconstituted product. Thrombin is included in the formulation at a concentration of 0.01-5.0 mg/mL, more commonly 0.1-5.0 mg/mL, and typically 0.5-3.0 mg/mL. Within certain embodiments of the invention the concentration of thrombin is 1.0 mg/mL.

The inventors have found that the ratio of mannitol to sucrose significantly affects protein unfolding during lyophilization. Mannitol is included as a bulking agent, to improve lyophilization cycle efficiency and lyophilized product appearance. Sucrose is included as a stabilizer (lyoprotectant), reducing thrombin unfolding during lyophilization and upon storage. Formulations having a mannitol:sucrose ratio of 5:1 (w/w) or greater were unacceptable; those having a ratio of 2.5:1 or less appeared stable as analyzed by Fourier transform infrared spectroscopy (FTIR). Ratios of 1:1 or less showed aggregation/precipitation in solution (upon reconstitution) as measured by right angle light scattering (RALS) and did not form an acceptable cake upon lyophilization. Thus, within the present invention the ratio of mannitol:sucrose will be greater than 1:1 but not greater than 2.5:1.

NaCl is included in the formulation to reduce aggregation and/or precipitation. As noted above, NaCl can be problematic in the lyophilization process. Within the formulation of the present invention, NaCl is included at a concentration of 50 mM to 300 mM. Within certain embodiments of the invention the concentration of NaCl is 75 mM to 250 mM, 100 mM to 200 mM, or 150 mM.

The thrombin formulation of the present invention is buffered to provide for stability during formulation and to optimize activity upon reconstitution. While not wishing to be bound by theory, it is believed that autolytic degradation of thrombin increases as pH increases above 6.0, and the precipitation rate increases as pH decreases below 6.0. It is therefore desirable to prepare thrombin at slightly acidic to approximately neutral pH to limit loss of activity due to autolysis (formation of inactive autolytic degradation products including β- and γ-thrombin). However, to provide optimal biological activity upon use, approximately physiological pH is desired. The present invention addresses these conflicting needs through the use of a weak buffer that is effective at slightly acidic to approximately neutral pH (i.e, pH 5.7-7.4), but will allow the pH to reach approximately physiological pH (i.e., pH 6.8 - 7.5, preferably pH 7.0 - 7.5, more preferably pH 7.35 - 7.45) when the thrombin is applied to bleeding tissue in a surgical setting. Within the present invention the thrombin solution is buffered at pH 5.7-7.4, commonly 6.0-6.5, and, within certain embodiments, 6.0. Suitable buffers include, in addition to histidine, other physiologically acceptable buffers that are effective within the noted pH range and will allow the solution to reach approximately physiological pH upon application of the composition in a surgical setting. Examples of such buffers include phosphate, citrate, Tris (2-Amino-2-(hydroxymethyl)-1,3-propanediol), and succinate. Within certain embodiments of the invention, histidine buffer is included at 1-20 mM, 2-10 mM, or 5 mM.

Suitable concentrations of other buffers within the present invention can be readily determined by one of ordinary skill in the art. Formulation buffers can be tested by adding blood and measuring the pH of the resulting solution. In an exemplary assay, aliquots of rabbit blood are added stepwise to various buffers, and pH is measured after each addition. When histidine buffers are tested in such an assay, 3.2 mM, 5 mM, 12.8 mM, and 20 mM histidine are neutralized by the addition of not more than 1.0 volume of blood. In contrast, 160 mM succinate buffer and 90 mM phophate/12.8 mM histidine buffer did not produce a mixture with a pH above 7.0 even after addition of 2-3 volumes of blood.

Within the present invention, a surfactant or high-molecular-weight polyethylene glycol (PEG) is included to prevent adsorptive losses and shear-induced thrombin aggregation/precipitation in solutions prior to lyophilization or after reconstitution of the lyophilized product. Suitable surfactants useful in this regard include polyethylene oxides, sorbitan esters, polyoxyethylene alkyl ethers, glycerides of fatty acids (e.g., glyceryl monooleate and glyceryl monostearate), polyoxyethylene sorbitan fatty acid esters (e.g., polysorbate 20 (polyoxyethylene sorbitan monolaurate) and polysorbate 80 (polyoxyethylene sorbitan monooleate)), and the like. High-molecular-weight polyethylene glycols include those having molecular weights from 400 to 8000, such as PEG 400, PEG 1000, PEG 3350, PEG 5000, and PEG 8000. An exemplary high-molecular-weight polyethylene glycol is PEG 3350. The preferred concentration of PEG 3350 is 0.1-1%. Although 0.03% PEG 3350 effectively inhibits adsorptive losses and shear-induced aggregation/precipitation, somewhat higher concentrations will commonly be used in the initial formulation to account for dilution following reconstitution. For example, a typical unit dose of thrombin can be prepared by filling a vial with 1.6 mL of a 1.0 mg/mL (3200 U/mg) solution (about 5000 NIH U/vial) and lyophilizing. The lyophilized product, when reconstituted with 5 mL of normal saline, provides a thrombin concentration of about 1000 NIH U/mL (0.32 mg/mL). If the concentration of PEG 3350 in the initial formulation is 0.1%, the concentration in the reconstituted product will be 0.03%.

Within certain embodiments of the invention, CaCl₂ is included in the formulation to promote hemostasis, as it is required to activate several blood clotting factors (e.g., Factor IX, Factor X). When included, the concentration of CaCl₂ in the formulation is up to 5 mM, generally 1-5 mM, commonly 4 mM. Inclusion of calcium chloride may also improve the physical stability of thrombin.

For long-term storage, the aqueous solution is aliquoted into sterile vials, ampoules, or other containers and lyophilized according to procedures known in the art. The lyophilized product appears as a powder or cake, a cake being the preferred form. The containers are then sealed. It is preferred to use a seal that permits later injection of diluent through the seal and into the container. The container is labeled according to standard practice in the pharmaceutical field.

In one embodiment of the invention, the container is provided in a kit with a second container containing a diluent. Suitable diluents include normal saline for injection and water for injection. The kit may further comprise an application device, such as a sprayer, syringe, or the like.

For use, the lyophilized thrombin composition is reconstituted with a suitable diluent to the desired concentration, generally from about 100 NIH U/ml to about 5,000 NIH U/ml, typically about 1,000 NIH U/ml, although the actual concentration will be determined by the physician according to the needs of the individual patient. The thrombin can be applied to bleeding tissue to achieve hemostasis, often in combination with an absorbable gelatin sponge. The thrombin can also be used as a component of a tissue adhesive or fibrin glue. These and other uses of thrombin are known in the art.

The invention is further illustrated by the following non-limiting examples.

### EXAMPLES

### Example 1

Recombinant human thrombin (rhThrombin) was formulated at 1 mg/ml in 5 mM histidine, 150 mM NaCl, 4 mM CaCl₂, 0.1% PEG3350, pH 6.0 with varying concentrations of mannitol and sucrose as shown in Table 2.

**Table 2**

| Formulation | Mannitol | Sucrose |
|---|---|---|
| T | 5% | 0.5% |
| A | 5% | 1% |
| B | 5% | 2% |
| C | 5% | 3% |
| D | 4% | 3% |
| E | 3% | 3% |
| F | -- | 5% |

1.6-ml aliquots of the solutions were placed in vials and lyophilized under the conditions shown in Table 3. For subsequent analysis, lyophilized samples were reconstituted with water where necessary. Visual observations of lyophilized and reconstituted samples are shown in Table 4.

**Table 3**

| Formulation | Freezing & Annealing | Primary Drying | Secondary Drying |
|---|---|---|---|
| T | 0.5°C/min to 5°C, hold 0.5 hr | 0.5 °C/min to -10°C, hold 16 hr | 0.2 °C/min to 30°C, hold 24 hr |
| | 0.25°C/min to -50°C, hold 2 hr | | |
| | 0.25°C/min to -30°C, hold 3 hr | 60 mTorr | 60 mTorr |
| | 0.25°C/min to -50°C, hold 2 hr | | |
| A, B, | 0.5°C/min to 5°C, hold 2 hr | 0.5 °C/min to -30 °C, | 0.5°C/min to 25°C, hold 24hr |
| C, D, | 0.5°C/min to -50°C, hold 2 hr | hold 10 hr | |
| E, F | 0.25°C/min to -20°C, hold 2 hr | 0.5 °C/min to -25 °C, hold 10 hr | 0.5°C/min to 30°C, hold 8 hr |
| | 0.25°C/min to -25°C, hold 2 hr | | |
| | 0.25°C/min to -50°C, hold 2 hr | 0.5 °C/min to -20 °C, hold 10 hr | 0.5°C/min to 20°C, hold 6 hr |
| | | 0.5 °C/min to -15 °C, hold 10 hr | 60 mTorr |
| | | 60 mTorr | |

**Table 4**

| Formulation | Lyophilized Solid | Reconstituted Solution |
|---|---|---|
| T | White cake | Clear |
| A | White cake | Clear |
| B | White cake | Clear |
| C | White cake | Clear |
| D | White cake | Clear |
| E | White semi-collapsed cake or white powder | Clear |
| F | White powder | Clear |

### Example 2

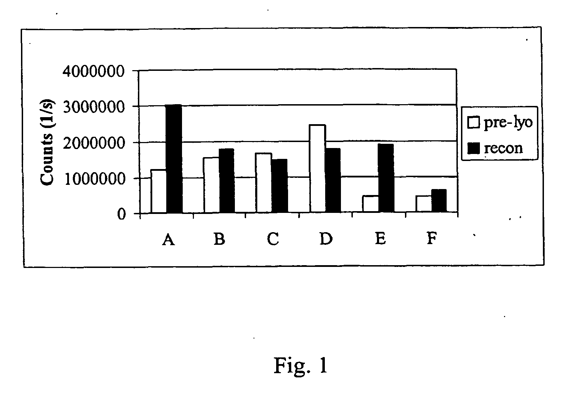

Formulations A, B, C, D, E, and F of rhThrombin were analyzed for the presence of aggregates/precipitants in solution. Pre- and post-lyophilization samples were analyzed by Right Angle Light Scattering (RALS) using a continuous spectrofluorometer (QUANTAMASTER QM4, Photon Technology International, Inc., Lawrenceville, NJ). The excitation and emission wavelengths were both set to 320 nm. Samples were loaded in a quartz vial of 1-cm pathlength. The slit widths were adjusted to 2 nm, and signals were collected at 1 point/sec for 60 seconds. The applied current was 75 watts. The reported values (see Fig. 1) were the average counts recorded over 60 seconds. Significant light scattering consistent with aggregation/precipitation after reconstitution was found in Formulation A. Formulations E and F did not produce an acceptable cake upon lyophilization.

### Example 3

Recovery of rhThrombin after lyophilization was measured using reverse-phase chromatography (RP-HPLC). Size-exclusion chromatography (SE-HPLC) was used to determine the percentage of soluble aggregate in test samples. Formulations A, B, C, D, E, and F were analyzed prior to lyophilization and after lyophilization and reconstitution.

For RP-HPLC, test samples and reference standard (rhThrombin, 1 mg/mL in dilution buffer (20 mM sodium phosphate, 140 mM NaCl, pH 7.0)) were filtered using centrifugal filter units (0.22 µm) (SPIN-X; Corning Life Sciences, Action, Mass.) at 14,000 RPM (20,800 RCF). A standard curve range from 5 to 40 µg was employed with samples injected at approximately 90% of the upper limit of quantification. Purity values were determined based on the area of the main peak relative to the total integrated peak area. Analysis was performed using an HPLC system (1100 Series; Agilent Technologies, Palo Alto, California) configured with:
- Binary pump with seal wash kit (G1312A)
- Four channel solvent degasser (G1322A)
- Thermostatted autosampler (G1329A/G1330A)
- Extended volume upgrade kit utilizing a 900 µL syringe (G1363A)
- Thermostatted column compartment with two heat exchangers (G1316A)
- Column switching upgrade kit (G1353A)
- Diode-array detector (G1315B) with 10 mm/13 µL flow cell
- Agilent CHEMSTATION Software (Revision A.08.03)

For SE-HPLC, test samples and reference standard were filtered using centrifugal filter units (0.22 µm) at 14,000 RPM (20,800 RCF). A standard curve range from 5.1 to 102 µg was utilized, with samples injected at approximately 90% of the upper limit of quantification. The percentage of aggregates was determined based on the area of the detected peaks preceding the main rhThrombin peak relative to the total integrated peak area. Analysis was performed using an HPLC system as disclosed above.

These analyses showed an increase in recovery of rhThrombin after lyophilization with increasing sucrose (≥3%) content as shown in Tables 5 and 6. Data are presented as rhThrombin concentration (mg/ml). The observation that increases in soluble aggregate did not necessarily correlate with lower recovery is consistent with the formation of insoluble rhThrombin aggregate during lyophilization.

**Table 5**

| RP-HPLC | | | | | | |
|---|---|---|---|---|---|---|
| Formulation: | A | B | C | D | E | F |
| Pre-lyophilization: | 1.03 | 1.06 | 1.02 | 1.04 | 1.03 | 1.04 |
| Reconstituted: | 0.91 | 0.92 | 0.93 | 0.94 | 0.96 | 0.96 |
| % Recovered | 88.3% | 86.8% | 91.2% | 90.4% | 93.2% | 92.3% |

**Table 6**

| % soluble aggregate by SE-HPLC | | | | | | |
|---|---|---|---|---|---|---|
| Formulation | A | B | C | D | E | F |
| Pre-lyophilization | 0.5 | 0.5 | 0.5 | 0.5 | 0.6 | 0.6 |
| Post-lyophilization | 0.6 | 0.7 | 0.7 | 0.7 | 0.5 | 0.5 |

### Example 4

Storage stability of rhThrombin formulations was studied over a 6-month period. Lyophilized samples of formulations A, B, C, D, E, and F were stored at 40°C and at 25°C and analyzed for content (thrombin concentration) by RP-HPLC at 0, 1, 2, 3, and 6 months as disclosed above. At both temperatures, formulation A, upon reconstitution, became turbid after two months (Table 7). Formulations E and F produced unacceptable cakes and were not analyzed after the 0-month time point. Stability, expressed as % recovery, is shown in Tables 8 and 9.

**Table 7**

| Temperature | Time | T | A | B | C | D |
|---|---|---|---|---|---|---|
| | 0 | Clear | Clear | Clear | Clear | Clear |
| | 1 month | Clear | Clear | Clear | Clear | Clear |
| -20°C | 2 months | Clear | Clear | Clear | Clear | Clear |
| | 3 months | Clear | Clear | Clear | Clear | Clear |
| | 6 months | Clear | Clear | Clear | Clear | Clear |
| | 1 week | Slightly turbid | Clear | Clear | Clear | Clear |
| | 2 week | Clear | Clear | Clear | Clear | Clear |
| | 1 month | Clear | Clear | Clear | Clear | Clear |
| 5°C | 2 months | Clear | Clear | Clear | Clear | Clear |
| | 3 months | Clear | Clear | Clear | Clear | Clear |
| | 6 months | Clear | Clear | Clear | Clear | Clear |
| | 1 week | Slightly turbid | Clear | Clear | Clear | Clear |
| | 2 week | Slightly turbid | Clear | Clear | Clear | Clear |
| 25°C | 1 month | Slightly turbid | Clear | Clear | Clear | Clear |
| | | | Slightly | | | |
| | 2 months | Clear | turbid | Clear | Clear | Clear |
| | | | Slightly | | | |
| | 3 months | Slightly turbid | turbid | Clear | Clear | Clear |
| | | | Slightly | | | |
| | 6 months | Clear | turbid | Clear | Clear | Clear |
| | 1 week | Slightly turbid | Not tested | Not tested | Not tested | Not tested |
| | 2 week | Slightly turbid | Not tested | Not tested | Not tested | Not tested |
| 40°C | 1 month | Slightly turbid | Clear | Clear | Clear | Clear |
| | 2 months | Not tested | Slightly turbid | Clear | Clear | Clear |
| | 3 months | Not tested | Slightly turbid | Clear | Clear | Clear |
| | 6 months | Not tested | Slightly turbid | Clear | Clear | Clear |

**Table 8**

| % recovery of thrombin during storage at 40 °C | | | | | | | |
|---|---|---|---|---|---|---|---|
| Formulation | Months | | | | | | |
| | 0 | 0.25 | 0.5 | 1 | 2 | 3 | 6 |
| T | 100 | 87.5 | 83.3 | 84.4 | n.t. | n.t. | n.t. |
| A | 100 | n.t. | n.t. | 95.6 | 94.5 | 92.3 | 90.1 |
| B | 100 | n.t. | n.t. | 96.7 | 98.9 | 95.7 | 92.4 |
| C | 100 | n.t. | n.t. | 96.8 | 95.7 | 93.5 | 91.4 |
| D | 100 | n.t. | n.t. | 97.9 | 97.9 | 95.7 | 93.6 |

| | | | | | | | |
|---|---|---|---|---|---|---|---|
| n.t. = not tested. | | | | | | | |

**Table 9**

| % recovery of thrombin during storage at 25 °C | | | | | | |
|---|---|---|---|---|---|---|
| Formulation | Months | | | | | |
| | 0 | 1 | 2 | 3 | 6 | 12 |
| Tox | 100 | 97.9 | 93.8 | 94.8 | 94.8 | 93.4 |
| A | 100 | 101 | 98.9 | 98.9 | 95.6 | 96.7 |
| B | 100 | 100 | 100 | 97.8 | 95.7 | 97.8 |
| C | 100 | 98.9 | 100 | 96.8 | n.t. | 100 |
| D | 100 | 98.9 | 98.9 | 97.9 | 97.9 | 97.9 |

| | | | | | | |
|---|---|---|---|---|---|---|
| *n.t. = not tested. | | | | | | |

### Example 5

Lyophilized samples of formulations A, B, C, D, E, and F were stored at 40°C and at 25°C and analyzed at 0, 1, 2, 3, and 6 months for the presence of high molecular weight impurities (soluble aggregate) by SE-HPLC as disclosed above. Results, shown in Table 10, indicated that in formulation T (containing the least amount of sucrose), increases in soluble aggregate were detected after one week. However, in formulations containing 1% or more sucrose, significant increases were not found after six months. Formulations E and F produced unacceptable cakes and were not analyzed after the 0-month time point.

**Table 10**

| % aggregates; storage at 40°C | | | | | | | |
|---|---|---|---|---|---|---|---|
| Formulation | Months | | | | | | |
| | 0 | 0.25 | 0.5 | 1 | 2 | 3 | 6 |
| T | 0.3 | 1.1 | 1.1 | 1.1 | n.t. | n.t. | n.t. |
| A | 0.6 | n.t. | n.t. | 0.7 | 0.8 | 0.6 | 0.8 |
| B | 0.7 | n.t | n.t. | 0.6 | 0.6 | 0.5 | 0.9 |
| C | 0.7 | n.t. | n.t. | 0.6 | 0.6 | 0.6 | 0.6 |
| D | 0.7 | n.t. | n.t. | 0.6 | 0.6 | 0.6 | 0.7 |

| | | | | | | | |
|---|---|---|---|---|---|---|---|
| n.t. = not tested. | | | | | | | |

**Table 11**

| % aggregates; storage at 25°C | | | | | | |
|---|---|---|---|---|---|---|
| Formulation | Months | | | | | |
| | 0 | 1 | 2 | 3 | 6 | 12 |
| T | 0.3 | 0.4 | 0.5 | 0.5 | 0.7 | 0.5 |
| A | 0.6 | 0.5 | 0.5 | 0.5 | 0.6 | 0.6 |
| B | 0.7 | 0.8 | 0.7 | 0.6 | 0.8 | 0.6 |
| C | 0.7 | 0.8 | 0.8 | 0.6 | 0.6 | 0.6 |
| D | 0.7 | 0.7 | 0.6 | 0.6 | 0.7 | 0.6 |

### Example 6

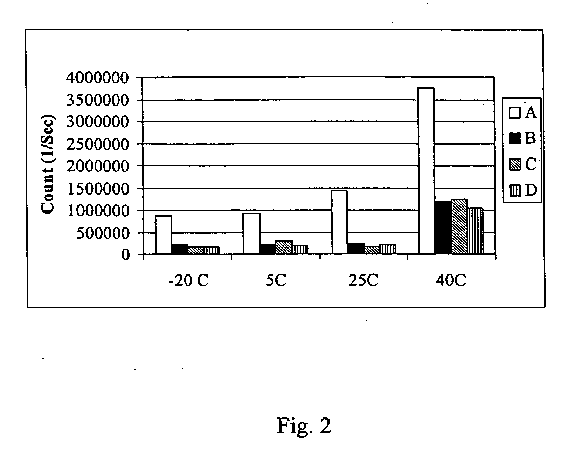

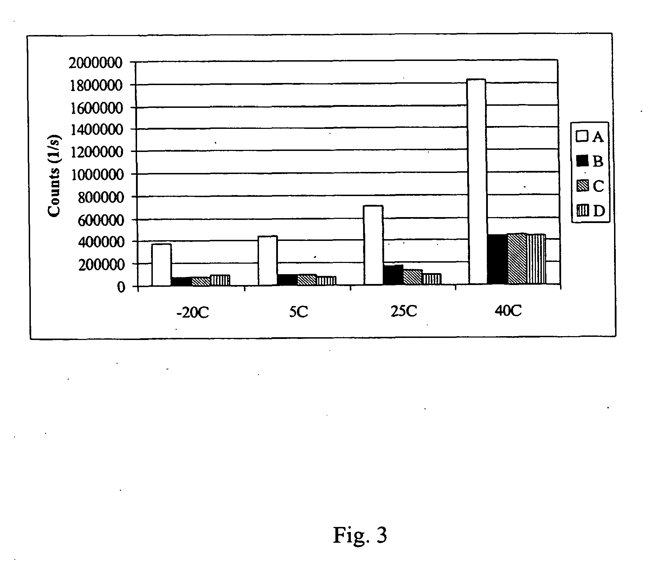

Lyophilized samples of formulations A, B, C, and D were stored for thirteen weeks and six months at -20°C, 5°C, 25°C and 40°C, then analyzed for RALS as disclosed in Example 2, above. Increases in light scatering measured by RALS are associated with the presence of insolubles and/or soluble aggregates. Results, shown in Tables 12 and 13 (as counts per second), and presented graphically in Figs. 2 and 3, indicated that Formulations B, C, and D were more stable than Formulation A with respect to aggregation during storage under all test conditions. Due to minor instrument variations between the 13-week and 6-month time points, the absolute numbers of two time points should not be compared; the RALS data should be compared only among the formulations tested at same time.

**Table 12**

| 13 weeks | | | | |
|---|---|---|---|---|
| Storage Temperature | Formulation | | | |
| | A | B | C | D |
| -20°C | 870,148.3 | 220,321.1 | 174,001.5 | 175,358.3 |
| 5°C | 917,635.8 | 209,274.9 | 298,378.7 | 183,227.1 |
| 25°C | 1,442,075 | 243,777.1 | 160,173.6 | 211,948.2 |
| 40°C | 3,746,816 | 1,196,508 | 1,245,188 | 1,049,673 |

**Table 13**

| 6 Months | | | | |
|---|---|---|---|---|
| Storage Temperature | Formulation | | | |
| | A | B | C | D |
| -20C | 377158.2 | 72443.12 | 74719.25 | 92018.51 |
| 5C | 438323.8 | 100675.1 | 100185.1 | 76518.18 |
| 25C | 705247.2 | 175997.6 | 134422.6 | 92118.5 |
| 40C | 1824263 | 438520.7 | 455521.4 | 446050.9 |

### Example 7

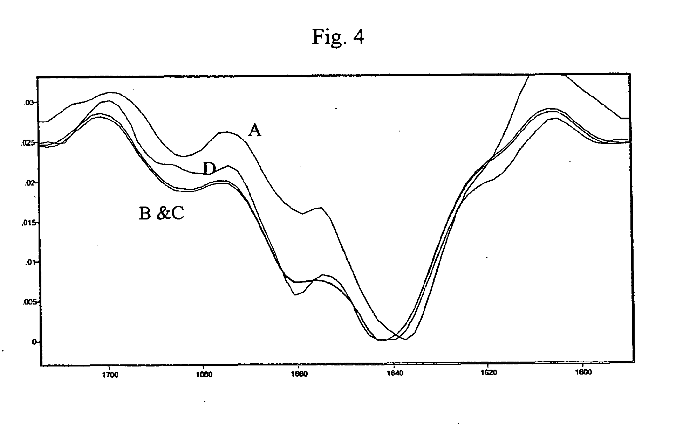

Lyophilized samples of formulations A, B, C, and D were analyzed by Fourier transform infrared (FTIR) spectroscopy to compare the secondary structure of rhThrombin in the lyophilized state. Infrared spectra were obtained with a FTIR spectrometer (FTLA2000-104, ABB Inc., Norwalk, Conn.). The lyophilized samples were mixed with potassium bromide (KBr) (approximate ratio 1:100 rhThrombin:KBr) and pressed to form pellets. The spectra were collected in single-beam transmission mode. To reduce the interferences from excipients and water vapor, the spectra of placebo and water vapor were subtracted from the protein spectra using analytical software (BOMEN-GRAMS/32 AI (Version 6.01); ABB Inc.). The second derivative spectra were created using the Savitzky-Golay function of second degree. The rhThrombin formulations were compared in the amide I region (1700 -1600 cm⁻¹). As shown in Fig. 4, similar spectra were obtained for formulations B, C, and D, with formulation A giving a different spectrum.

Three replicates of formulation A were then tested by FTIR as disclosed above. The resulting spectra were essentially identical.

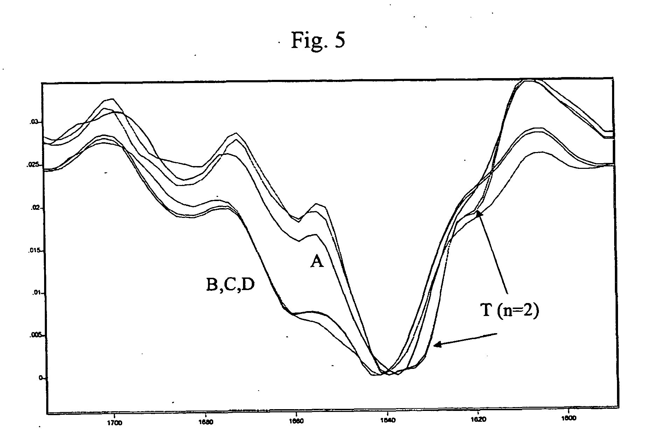

Formulations A, B, C, and D were then compared to a formulation containing 5% mannitol and 0.5% sucrose (Formulation T). FTIR was carried out as disclosed above. As shown in Fig. 5, Formulations B, C, and D gave similar spectra, while the spectra of formulations containing 5% mannitol and either 1% sucrose or 0.5% sucrose indicated a different spectra. The increases in RALS observed after lyophilization when formulations containing ≤1% sucrose are compared to those containing ≥2% sucrose are consistent with these findings (Example 6). These data suggest that thrombin is partially unfolded in formulations containing ≤1% sucrose (leading to increased insolubles upon reconstitution), whereas, the secondary structure of thrombin is stabilized in the presence of ≥2% sucrose during lyophilization.

### Further embodiments of the invention

1. A composition consisting essentially of:
   0.1 mg/mL to 5.0 mg/mL thrombin;
   2% to 4% (w/v) sucrose;
   3.5% to 5% (w/v) mannitol;
   50 mM to 300 mM NaCl;
   0-5mM CaCl_{2;}
   0.03% to 1% (w/v) of a surfactant or high-molecular-weight polyethylene glycol; and
   a physiologically acceptable buffer,
   in aqueous solution at pH 5.7 - 7.4, wherein concentration of the buffer is selected to provide approximately physiological pH upon application of the composition in a surgical setting and wherein the ratio of mannitol:sucrose is greater than 1:1 but not greater than 2.5:1 (w/w).
2. The composition of embodiment 1 wherein the ratio of mannitol to sucrose is 1.33:1 (w/w).
3. The composition of embodiment 1 wherein the molar ratio of sucrose:thrombin is at least 700:1.
4. The composition of embodiment 1 wherein the molar ratio of sucrose:thrombin is at least 2000:1.
5. The composition of embodiment 1 wherein the thrombin is human thrombin.
6. The composition of embodiment 1 wherein the thrombin is recombinant human thrombin.
7. The composition of embodiment 1 wherein the thrombin concentration is 0.5 - 3.0 mg/ml.
8. The composition of embodiment 1 wherein the thrombin concentration is 1 mg/ml.
9. The composition of embodiment 1 wherein the sucrose concentration is 3% (w/v).
10. The composition of embodiment 1 wherein the mannitol concentration is 4% (w/v).
11. The composition of embodiment 1 wherein the sucrose concentration is 3% (w/v) and the mannitol concentration is 4% (w/v).
12. The composition of embodiment 1 wherein the surfactant or high-molecular-weight polyethylene glycol is PEG 3350.
13. The composition of embodiment 12 wherein the concentration of PEG 3350 is 0.1 % (w/v).
14. The composition of embodiment 1 wherein the pH is 6.0.
15. The composition of embodiment 1 wherein the buffer is selected from the group consisting of histidine, citrate, phosphate, Tris, and succinate buffers.
16. The composition of embodiment 1 wherein the buffer is histidine buffer.
17. The composition of embodiment 16 wherein the histidine buffer concentration is 2.0-10 mM.
18. The composition of embodiment 1 wherein the buffer is 5 mM histidine pH 6.0.
19. The thrombin composition of embodiment 1, consisting essentially of:
   0.1 mg/mL to 5.0 mg/mL thrombin;
   2% to 4% (w/v) sucrose;
   3.5% to 5% (w/v) mannitol;
   100 mM to 200 mM NaCl;
   1 mM to 5 mM CaCl₂;
   0.03% to 1% (w/v) PEG3350; and
   2 mM to 10 mM histidine
   in aqueous solution at pH 5.5 - 6.5, wherein the ratio of mannitol:sucrose is greater than 1:1 but not greater than 2.5:1 (w/w).
20. The thrombin composition of embodiment 19, consisting essentially of:
   1 mg/ml thrombin;
   3% (w/v) sucrose;
   4% (w/v) mannitol;
   4 mM CaCl₂;
   0.1% (w/v) PEG3350;
   150 mM NaCl; and
   5 mM histidine
   in aqueous solution at pH 6.0.
21. A method of preparing a lyophilized thrombin composition comprising:
   providing a composition according to embodiment 1; and
   lyophilizing the aqueous solution to form a lyophilized thrombin composition.
22. The method of embodiment 21 wherein, within the providing step, the composition consists essentially of:
   0.1 mg/mL to 5.0 mg/mL thrombin;
   2% to 4% (w/v) sucrose;
   3.5% to 5% (w/v) mannitol;
   100 mM to 200 mM NaCl;
   1 mM to 5 mM CaCl₂;
   0.03% to 1% (w/v) PEG3350; and
   2 mM to 10 mM histidine
   in aqueous solution at pH 5.5 - 6.5, wherein the ratio of mannitol:sucrose is greater than 1:1 but not greater than 2.5:1 (w/w).
23. A lyophilized thrombin composition prepared by the method of embodiment 21.
24. The composition of embodiment 23 contained in a sealed container having a label affixed to an exterior surface thereof.
25. The composition of embodiment 24, which contains from 2,500 to 20,000 NIH units of thrombin.
26. A kit comprising the composition of embodiment 24 and a second sealed container containing a diluent.
27. The kit of embodiment 26 wherein the diluent is normal saline.

## Claims

1. A lyophilized composition prepared from a solution consisting essentially of recombinant human thrombin, sucrose, mannitol, NaCl, CaCl₂, a surfactant or high-molecular-weight polyethylene glycol, and a physiologically acceptable buffer,
wherein the concentration of said recombinant human thrombin in said solution is from 0.1 mg/mL to 5.0 mg/mL;
the concentration of said sucrose in said solution is from 2% to 4% (w/v);
the concentration of said mannitol in said solution is from 3.5% to 5% (w/v);
the concentration of said NaCl in said solution is from 50 mM to 300 mM;
the concentration of said CaCl₂ in said solution is from 0mM to 5mM; and
the concentration of said surfactant or high-molecular-weight polyethylene glycol in said solution is from 0.03% to 1% (w/v),
wherein said solution has a pH from 5.7 to 7.4 and provides approximately physiological pH upon application of the composition in a surgical setting, and wherein said solution has a ratio of mannitol: sucrose that is greater than 1:1 bust not greater than 2.5:1 (w/w).

2. A lyophilized composition prepared from a solution consisting essentially of recombinant human thrombin, sucrose, mannitol, NaCl, CaCl₂, PEG 3350, and a histidine buffer,
wherein the concentration of said recombinant human thrombin in said solution is from 0.1 mg/mL to 5.0 mg/mL;
the concentration of said sucrose in said solution is from 2% to 4% (w/v);
the concentration of said mannitol in said solution is from 3.5% to 5% (w/v);
the concentration of said NaCl in said solution is from 100 mM to 200 mM;
the concentration of said CaCl₂ in said solution is from 1mM to 5mM;
the concentration of said PEG 3350 in said solution is from 0.03% to 1% (w/v); and
the concentration of said histidine in said solution is from 2mM to 10mM;
wherein said solution has a pH from 5.5 to 6.5 and provides approximately physiological pH upon application of the composition in a surgical setting, and wherein said solution has a ratio of mannitol: sucrose that is greater than 1:1 but not greater than 2.5:1 (w/w).

3. A lyophilized composition according to claim 2 prepared from a solution at pH 6.0 comprising 1 mg/mL recombinant human thrombin in 5mM histidine, 2% sucrose, 5% mannitol, 150mM NaCl, 4mM CaCl₂, and 0.1% PEG 3350.

4. A lyophilized composition according to claim 2 prepared from a solution at pH 6.0 comprising 1 mg/mL recombinant human thrombin in 5mM histidine, 3% sucrose, 5% mannitol, 150mM NaCl, 4mM CaCl₂, and 0.1% PEG 3350.

5. A lyophilized composition according to claim 2 prepared from a solution at pH 6.0 comprising 1 mg/mL recombinant human thrombin in 5mM histidine, 3% sucrose, 4% mannitol, 150mM NaCl, 4mM CaCl₂, and 0.1% PEG 3350.

6. The lyophilized composition of any claim 1 or 2 wherein said ratio of mannitol to sucrose is 1.33:1 (w/w).

7. The lyophilized composition of any preceding claim wherein said solution has a molar ratio of sucrose:thrombin that is at least 700:1.

8. The lyophilized composition of any preceding claim, wherein said solution has a molar ratio of sucrose: thrombin that is at least 2000:1.

9. The lyophilized composition of claim 1 or 2, wherein said solution has a recombinant human thrombin concentration that is from 0.5 to 3.0 mg/mL.

10. The lyophilized composition of claim 1 or 2 wherein said solution has a recombinant human thrombin concentration that is 1 mg/ml.

11. The lyophilized composition of claim 1 or 2 wherein said solution has a sucrose concentration that is 3% (w/v).

12. The lyophilized composition of claim 1 or 2 wherein said solution has a mannitol concentration that is 4% (w/v).

13. The lyophilized composition of claim 1 or 2, wherein said solution has a sucrose concentration that is 3% (w/v) and a mannitol concentration that is 4% (w/v).

14. The lyophilized composition of claim 1 or 2, wherein said solution has a concentration of PEG 3350 that is 0.1% (w/v).

15. The lyophilized composition of claim 1 or 2, wherein the pH of said solution is 6.0.

16. The lyophilized composition of claim 1, wherein said physiologically acceptable buffer is selected from the group consisting of histidine, citrate, phosphate, Tris, and succinate buffers.

17. The lyophilized composition of claim 1, wherein said physiologically acceptable buffer is a histidine buffer.

18. The lyophilized composition of claim 17, wherein said histidine buffer concentration in said solution is from 2.0 to 10 mM.

19. The lyophilized composition of claim 1 wherein said physiologically acceptable buffer in said solution is 5 mM histidine at pH 6.0.

20. A kit comprising a sealed container containing said lyophilized composition of any of the preceding claims.

21. A kit according to claim 20 wherein the sealed container contains from 2500 NIH units to 20000 NIH units recombinant human thrombin.

22. The kit of claim 20 or 21 further comprising a second sealed container containing a diluent.

23. The kit of claim 22 wherein said diluent is normal saline.

24. Use of a composition in the preparation of a medicament for treatment of a bleeding tissue, wherein said composition is prepared from an aqueous solution consisting essentially of: thrombin, sucrose, mannitol, NaCl, CaCl₂, a surfactant or high-molecular-weight polyethylene glycol, and a physiologically acceptable buffer,
wherein the concentration of said thrombin in said solution is from 0.1 mg/mL to 5.0 mg/mL;
the concentration of said sucrose in said solution is from 2% to 4% (w/v);
the concentration of said mannitol in said solution is from 3.5% to 5% (w/v);
the concentration of said NaCl in said solution is from 50 mM to 300 mM;
the concentration of said CaCl₂ in said solution is from 0 mM to 5 mM; and
the concentration of said surfactant or high-molecular-weight polyethylene glycol in said solution is from 0.03% to 1% (w/v),
wherein said solution has a pH from 5.7 to 7.4 and provides approximately physiological pH upon application of the composition in a surgical setting, and wherein said solution has a ratio of mannitol: sucrose that is greater than 1:1 but not greater than 2.5:1 (w/w); and
wherein the preparation of the medicament includes the steps of lyophilizing the aqueous solution to form a lyophilized thrombin composition and reconstituting the lyophilized thrombin composition with a suitable diluent.

25. Use according to claim 24 wherein the medicament comprises a tissue adhesive or fibrin glue.

26. Use according to claim 24 wherein the treatment of a bleeding tissue comprises use of an absorbable gelatin sponge.

27. Use according to claim 24 wherein the reconstituted thrombin composition comprises thrombin at 100 NIH U/ml to 5000 NIH U/ml.

28. The lyophilized composition according to any of claims 1-19 for use in a method of achieving hemostasis, wherein the method includes reconstitution of the composition with a suitable diluent and application to a bleeding tissue.

29. A composition according to claim 28 wherein the application to bleeding tissue is in combination with an absorbable gelatin sponge.

30. A composition according to claim 28 wherein the composition is a component of a tissue adhesive or fibrin glue.

31. A composition according to claim 28 wherein the reconstituted thrombin composition comprises thrombin at 100 NIH U/ml to 5000 NIH U/ml.

## Patentansprüche

1. Gefriergetrocknete Zusammensetzung, hergestellt aus einer Lösung, die im Wesentlichen aus rekombinantem humanem Thrombin, Saccharose, Mannitol, NaCl, CaCl₂, einem Tensid oder hochmolekularem Polyethylenglycol und einem physiologisch akzeptierbaren Puffer besteht,
wobei die Konzentration des rekombinanten humanen Thrombins in der Lösung 0,1 mg/ml bis 5,0 mg/ml beträgt;
die Konzentration der Saccharose in der Lösung 2 bis 4 % (m/V) beträgt;
die Konzentration des Mannitols in der Lösung 3,5 bis 5 % (m/V) beträgt;
die Konzentration des NaCl in der Lösung 50 mM bis 300 mM beträgt;
die Konzentration des CaCl₂ in der Lösung 0 mM bis 5 mM beträgt; und
die Konzentration des Tensids oder des hochmolekularen Polyethylenglycols in der Lösung 0,03 bis 1 % (m/V) beträgt,
wobei die Lösung einen pH-Wert von 5,7 bis 7,4 aufweist und bei Anwendung der Zusammensetzung im chirurgischen Rahmen einen annähernd physiologischen pH-Wert bereitstellt, und wobei die Lösung ein Mannitol:Saccharose-Verhältnis von mehr als 1:1, aber nicht mehr als 2,5:1 (m/m) aufweist.

2. Gefriergetrocknete Zusammensetzung, hergestellt aus einer Lösung, die im Wesentlichen aus rekombinantem humanem Thrombin, Saccharose, Mannitol, NaCl, CaCl₂, PEG 3350 und einem Histidin-Puffer besteht,
wobei die Konzentration des rekombinanten humanen Thrombins in der Lösung 0,1 mg/ml bis 5,0 mg/ml beträgt;
die Konzentration der Saccharose in der Lösung 2 bis 4 % (m/V) beträgt;
die Konzentration des Mannitols in der Lösung 3,5 bis 5 % (m/V) beträgt;
die Konzentration des NaCl in der Lösung 100 mM bis 200 mM beträgt;
die Konzentration des CaCl₂ in der Lösung 1 mM bis 5 mM beträgt; und
die Konzentration des PEG 3350 in der Lösung 0,03 bis 1 % (m/V) beträgt; und die Konzentration des Histidins in der Lösung 2 mM bis 10 mM beträgt;
wobei die Lösung einen pH-Wert von 5,5 bis 6,5 aufweist und bei Anwendung der Zusammensetzung im chirurgischen Rahmen einen annähernd physiologischen pH-Wert bereitstellt, und wobei die Lösung ein Mannitol:Saccharose-Verhältnis von mehr als 1:1, aber nicht mehr als 2,5:1 (m/m) aufweist.

3. Gefriergetrocknete Zusammensetzung nach Anspruch 2, hergestellt aus einer Lösung mit dem pH-Wert 6,0, die 1 mg/ml rekombinantes humanes Thrombin in 5 mM Histidin, 2 % Saccharose, 5 % Mannitol, 150 mM NaCl, 4 mM CaCl₂ und 0,1 % PEG 3350 aufweist.

4. Gefriergetrocknete Zusammensetzung nach Anspruch 2, hergestellt aus einer Lösung mit dem pH-Wert 6,0, die 1 mg/ml rekombinantes humanes Thrombin in 5 mM Histidin, 3 % Saccharose, 5 % Mannitol, 150 mM NaCI, 4 mM CaCl₂ und 0,1 % PEG 3350 aufweist.

5. Gefriergetrocknete Zusammensetzung nach Anspruch 2, hergestellt aus einer Lösung mit dem pH-Wert 6,0, die 1 mg/ml rekombinantes humanes Thrombin in 5 mM Histidin, 3 % Saccharose, 4 % Mannitol, 150 mM NaCl, 4 mM CaCl₂ und 0,1 % PEG 3350 aufweist.

6. Gefriergetrocknete Zusammensetzung nach Anspruch 1 oder 2, wobei das % Verhältnis von Mannitol zu Saccharose gleich 1,33:1 (m/m) ist.

7. Gefriergetrocknete Zusammensetzung nach einem der vorstehenden Ansprüche, wobei die Lösung ein Molverhältnis von Saccharose zu Thrombin aufweist, das mindestens gleich 700:1 ist.

8. Gefriergetrocknete Zusammensetzung nach einem der vorstehenden Ansprüche, wobei die Lösung ein Molverhältnis von Saccharose zu Thrombin aufweist, das mindestens gleich 2000:1 ist.

9. Gefriergetrocknete Zusammensetzung nach Anspruch 1 oder 2, wobei die Lösung eine Konzentration des rekombinanten humanen Thrombins von 0,5 bis 3,0 mg/ml aufweist.

10. Gefriergetrocknete Zusammensetzung nach Anspruch 1 oder 2, wobei die Lösung eine Konzentration des rekombinanten humanen Thrombins von 1 mg/ml aufweist.

11. Gefriergetrocknete Zusammensetzung nach Anspruch 1 oder 2, wobei die Lösung eine Saccharosekonzentration von 3 % (m/V) aufweist.

12. Gefriergetrocknete Zusammensetzung nach Anspruch 1 oder 2, wobei die Lösung eine Mannitolkonzentration von 4 % (m/V) aufweist.

13. Gefriergetrocknete Zusammensetzung nach Anspruch 1 oder 2, wobei die Lösung eine Saccharosekonzentration von 3 % (m/V) und eine Mannitolkonzentration von 4 % (m/V) aufweist.

14. Gefriergetrocknete Zusammensetzung nach Anspruch 1 oder 2, wobei die Lösung eine PEG 3350-Konzentration von 0,1 % (m/V) aufweist.

15. Gefriergetrocknete Zusammensetzung nach Anspruch 1 oder 2, wobei der pH-Wert der Lösung 6,0 ist.

16. Gefriergetrocknete Zusammensetzung nach Anspruch 1, wobei der physiologisch akzeptierbare Puffer ausgewählt ist aus Histidin-, Citrat-, Phosphat-, Tris- und Succinat-Puffern besteht.

17. Gefriergetrocknete Zusammensetzung nach Anspruch 1, wobei der physiologisch akzeptierbare Puffer ein Histidin-Puffer ist.

18. Gefriergetrocknete Zusammensetzung nach Anspruch 17, wobei die Konzentration des Histidin-Puffers in der Lösung 2,0 bis 10 mM beträgt.

19. Gefriergetrocknete Zusammensetzung nach Anspruch 1, wobei der physiologisch akzeptierbare Puffer in der Lösung 5 mM Histidin bei pH 6,0 ist.

20. Kit, der einen versiegelten Behälter aufweist, der die gefriergetrocknete Zusammensetzung nach einem der vorstehenden Ansprüche enthält.

21. Kit nach Anspruch 20, wobei der versiegelte Behälter 2500 NIH-Einheiten bis 20000 NIH-Einheiten rekombinantes humanes Thrombin enthält.

22. Kit nach Anspruch 20 oder 21, der ferner einen zweiten versiegelten Behälter aufweist, der ein Verdünnungsmittel enthält.

23. Kit nach Anspruch 22, wobei das Verdünnungsmittel physiologische Kochsalzlösung ist.

24. Verwendung einer Zusammensetzung bei der Herstellung eines Medikaments zur Behandlung eines blutenden Gewebes, wobei die Zusammensetzung aus einer wässrigen Lösung hergestellt wird, die im Wesentlichen aus Thrombin, Saccharose, Mannitol, NaCl, CaCl₂, einem Tensid oder hochmolekularen Polyethylenglycol und einem physiologisch akzeptierbaren Puffer besteht,
wobei die Konzentration des Thrombins in der Lösung 0,1 mg/ml bis 5,0 mg/ml beträgt;
die Konzentration der Saccharose in der Lösung 2 bis 4 % (m/V) beträgt;
die Konzentration des Mannitols in der Lösung 3,5 bis 5 % (m/V) beträgt;
die Konzentration des NaCl in der Lösung 50 mM bis 300 mM beträgt;
die Konzentration des CaCl₂ in der Lösung 0 mM bis 5 mM beträgt; und
die Konzentration des Tensids oder des hochmolekularen Polyethylenglycols in der Lösung 0,03 bis 1 % (m/V) beträgt,
wobei die Lösung einen pH-Wert von 5,7 bis 7,4 aufweist und bei Anwendung der Zusammensetzung im chirurgischen Rahmen einen annähernd physiologischen pH-Wert bereitstellt, und wobei die Lösung ein Mannitol:Saccharose-Verhältnis von mehr als 1:1, aber nicht mehr als 2,5:1 (m/m) aufweist; und
wobei die Herstellung des Medikaments die Schritte des Gefriertrocknens der wässrigen Lösung zur Bildung einer gefriergetrockneten Thrombinzusammensetzung und des Verdünnens der gefriergetrockneten Thrombinzusammensetzung zur ursprünglichen Konzentration mit einem geeigneten Verdünnungsmittel aufweist.

25. Verwendung nach Anspruch 24, wobei das Medikament einen Gewebekleber oder Fibrinkleber aufweist.

26. Verwendung nach Anspruch 24, wobei die Behandlung eines blutenden Gewebes die Anwendung einer absorbierbaren Gelatineschwamms aufweist.

27. Verwendung nach Anspruch 24, wobei die zur ursprünglichen Konzentration verdünnte Thrombinzusammensetzung 100 NIH-Einheiten/ml bis 5000 NIH-Einheiten/ml Thrombin aufweist.

28. Gefriergetrocknete Zusammensetzung nach einem der Ansprüche 1-19 zur Verwendung bei einem Verfahren zum Erreichen von Hämostase, wobei das Verfahren das Verdünnen der Zusammensetzung zur ursprünglichen Konzentration mit einem geeigneten Verdünnungsmittel und Auftragen auf ein blutendes Gewebe beinhaltet.

29. Zusammensetzung nach Anspruch 28, wobei der Auftrag auf blutendes Gewebe in Verbindung mit einem absorbierbaren Gelatineschwamm erfolgt.

30. Zusammensetzung nach Anspruch 28, wobei die Zusammensetzung eine Komponente eines Gewebeklebers oder Fibrinklebers ist.

31. Zusammensetzung nach Anspruch 28, wobei die zur ursprünglichen Konzentration verdünnte Thrombinzusammensetzung 100 NIH-Einheiten/ml bis 5000 NIH-Einheiten/ml Thrombin aufweist.

## Revendications

1. Composition lyophilisée préparée à partir d'une solution constituée essentiellement de thrombine humaine recombinante, de saccharose, de mannitol, de NaCl, de CaCl₂, d'un tensioactif ou d'un polyéthylèneglycol à haut poids moléculaire, et d'un tampon physiologiquement acceptable,
dans laquelle la concentration de ladite thrombine humaine recombinante dans ladite solution est de 0,1 mg/ml à 5,0 mg/ml;
la concentration dudit saccharose dans ladite solution est de 2% à 4% (p/v);
la concentration dudit mannitol dans ladite solution est de 3,5% à 5% (p/v);
la concentration dudit NaCl dans ladite solution est de 50 mM à 300 mM;
la concentration dudit CaCl₂ dans ladite solution est de 0 mM à 5 mM; et
la concentration dudit tensioactif ou polyéthylèneglycol à haut poids moléculaire dans ladite solution est de 0,03% à 1% (p/v),
dans laquelle ladite solution possède un pH de 5,7 à 7,4 et donne un pH approximativement physiologique lors d'une application de la composition dans un arrangement chirurgical et dans laquelle ladite solution possède un rapport de mannitol:saccharose qui est supérieur à 1:1 mais pas supérieur à 2,5:1 (p/p).

2. Composition lyophilisée préparée à partir d'une solution constituée essentiellement de thrombine humaine recombinante, de saccharose, de mannitol, de NaCl, de CaCl₂, de PEG 3350, et d'un tampon d'histidine,
dans laquelle la concentration de ladite thrombine humaine recombinante dans ladite solution est de 0,1 mg/ml à 5,0 mg/ml;
la concentration dudit saccharose dans ladite solution est de 2% à 4% (p/v);
la concentration dudit mannitol dans ladite solution est de 3,5% à 5% (p/v);
la concentration dudit NaCl dans ladite solution est de 100 mM à 200 mM;
la concentration dudit CaCl₂ dans ladite solution est de 1 mM à 5 mM;
la concentration dudit PEG 3350 dans ladite solution est de 0,03% à 1% (p/v); et
la concentration de ladite histidine dans ladite solution est de 2 mM à 10 mM;
dans laquelle ladite solution possède un pH de 5,5 à 6,5 et donne un pH approximativement physiologique lors d'une application de la composition dans un arrangement chirurgical et dans laquelle ladite solution possède un rapport de mannitol:saccharose qui est supérieur à 1:1 mais pas supérieur à 2,5:1 (p/p).

3. Composition lyophilisée selon la revendication 2 préparée à partir d'une solution à pH 6,0 comprenant 1 mg/ml de thrombine humaine recombinante dans 5 mM d'histidine, 2% de saccharose, 5% de mannitol, 150 mM de NaCl, 4mM de CaCl₂ et 0,1% de PEG 3350.

4. Composition lyophilisée selon la revendication 2 préparée à partir d'une solution à pH 6,0 comprenant 1 mg/ml de thrombine humaine recombinante dans 5 mM d'histidine, 3% de saccharose, 5% de mannitol, 150 mM de NaCl, 4 mM de CaCl₂ et 0,1% de PEG 3350.

5. Composition lyophilisée selon la revendication 2 préparée à partir d'une solution à pH 6,0 comprenant 1 mg/ml de thrombine humaine recombinante dans 5 mM d'histidine, 3% de saccharose, 4% de mannitol, 150 mM de NaCl, 4 mM de CaCl₂ et 0,1% de PEG 3350.

6. Composition lyophilisée selon l'une quelconque de la revendication 1 ou 2, dans laquelle ledit rapport de mannitol sur saccharose est de 1,33:1 (p/p).

7. Composition lyophilisée selon l'une quelconque des revendications précédentes, dans laquelle ladite solution possède un rapport molaire de saccharose:thrombine qui est d'au moins 700:1.

8. Composition lyophilisée selon l'une quelconque des revendications précédentes, dans laquelle ladite solution possède un rapport molaire de saccharose:thrombine qui est d'au moins 2000:1.

9. Composition lyophilisée selon la revendication 1 ou 2, dans laquelle ladite solution possède une concentration de thrombine humaine recombinante qui est de 0,5 à 3,0 mg/ml.

10. Composition lyophilisée selon la revendication 1 ou 2, dans laquelle ladite solution possède une concentration de thrombine humaine recombinante qui est de 1 mg/ml.

11. Composition lyophilisée selon la revendication 1 ou 2, dans laquelle ladite solution possède une concentration de saccharose qui est de 3% (p/v).

12. Composition lyophilisée selon la revendication 1 ou 2, dans laquelle ladite solution possède une concentration de mannitol qui est de 4% (p/v).

13. Composition lyophilisée selon la revendication 1 ou 2, dans laquelle ladite solution possède une concentration de saccharose qui est de 3% (p/v) et une concentration de mannitol qui est de 4% (p/v).

14. Composition lyophilisée selon la revendication 1 ou 2, dans laquelle ladite solution possède une concentration de PEG 3350 qui est de 0,1% (p/v).

15. Composition lyophilisée selon la revendication 1 ou 2, dans laquelle le pH de ladite solution est de 6,0.

16. Composition lyophilisée selon la revendication 1, dans laquelle ledit tampon physiologiquement acceptable est choisi dans le groupe constitué de tampons d'histidine, de citrate, de phosphate, de Tris et de succinate.

17. Composition lyophilisée selon la revendication 1, dans laquelle ledit tampon physiologiquement acceptable est un tampon d'histidine.

18. Composition lyophilisée selon la revendication 17, dans laquelle la concentration dudit tampon d'histidine dans ladite solution est de 2,0 à 10 mM.

19. Composition lyophilisée selon la revendication 1, dans laquelle ledit tampon physiologiquement acceptable dans ladite solution est 5 mM d'histidine à pH 6,0.

20. Kit comprenant un récipient scellé contenant ladite composition lyophilisée selon l'une quelconque des revendications précédentes.

21. Kit selon la revendication 20, dans lequel le récipient scellé contient de 2500 unités NIH à 20000 unités NIH de thrombine humaine recombinante.

22. Kit selon la revendication 20 ou 21, comprenant en outre un deuxième récipient scellé contenant un diluant.

23. Kit selon la revendication 22, dans lequel ledit diluant est une solution saline normale.

24. Utilisation d'une composition dans la préparation d'un médicament pour traitement d'un tissu qui saigne, dans laquelle ladite composition est préparée à partir d'une solution aqueuse constituée essentiellement de: thrombine, saccharose, mannitol, NaCl, CaCl₂, un tensioactif ou un polyéthylèneglycol à haut poids moléculaire, et un tampon physiologiquement acceptable,
dans laquelle la concentration de ladite thrombine dans ladite solution est de 0,1 mg/ml à 5,0 mg/ml;
la concentration dudit saccharose dans ladite solution est de 2% à 4% (p/v);
la concentration dudit mannitol dans ladite solution est de 3,5% à 5% (p/v);
la concentration dudit NaCl dans ladite solution est de 50 mM à 300 mM;
la concentration dudit CaCl₂ dans ladite solution est de 0 mM à 5 mM; et
la concentration dudit tensioactif ou polyéthylèneglycol à haut poids moléculaire dans ladite solution est de 0,03% à 1% (p/v),
dans laquelle ladite solution possède un pH de 5,7 à 7,4 et donne un pH approximativement physiologique lors d'une application de la composition dans un arrangement chirurgical et dans laquelle ladite solution possède un rapport de mannitol:saccharose qui est supérieur à 1:1 mais pas supérieur à 2,5:1 (p/p); et
dans laquelle la préparation du médicament inclut les étapes de lyophilisation de la solution aqueuse pour former une composition de thrombine lyophilisée et de reconstitution de la composition de thrombine lyophilisée avec un diluant approprié.

25. Utilisation selon la revendication 24, dans laquelle le médicament comprend un adhésif tissulaire ou une colle de fibrine.

26. Utilisation selon la revendication 24, dans laquelle le traitement d'un tissu qui saigne comprend l'utilisation d'une éponge de gélatine absorbable.

27. Utilisation selon la revendication 24, dans laquelle la composition de thrombine reconstituée comprend de la thrombine à 100 NIH U/ml à 5000 NIH U/ml.

28. Composition lyophilisée selon l'une quelconque des revendications 1-19 pour une utilisation dans une méthode d'obtention d'une hémostase, dans laquelle la méthode inclut la reconstitution de la composition avec un diluant approprié et l'application sur un tissu qui saigne.

29. Composition selon la revendication 28, dans laquelle l'application sur un tissu qui saigne est en combinaison avec une éponge de gélatine absorbable.

30. Composition selon la revendication 28, où la composition est une composante d'un adhésif tissulaire ou d'une colle de fibrine.

31. Composition selon la revendication 28, dans laquelle la composition de thrombine reconstituée comprend de la thrombine à 100 NIH U/ml à 5000 NIH U/ml.
